# EUROPEAN PATENT SPECIFICATION

(11) **EP 2 456 758 B1**
(45) Date of publication and mention of the grant of the patent: **06.08.2014**
(21) Application number: 11700054.7
(22) Date of filing: 05.01.2011
(51) Int. Cl.: C07D 213/12, A61K 8/49

(54) **A PROCESS FOR PREPARING TRISUBSTITUTED PHENYL DERIVATIVES COMPRISING A (1H-1,2,4-TRIAZOL-1-YL)ALKYL GROUP**
VERFAHREN ZUR HERSTELLUNG DREIFACH SUBSTITUIERTER PHENYLDERIVATE, DIE EINE (1H-1,2,4-TRIAZOL-1-YL)ALKYL-GRUPPE UMFASSEN
PROCÉDÉ DE PRÉPARATION DE DÉRIVÉS DE PHÉNYLE TRISUBSTITUÉ COMPRENANT UN GROUPE (1H-1,2,4-TRIAZOL-1-YL)ALKYLE

(30) Priority: 07.01.2010 EP 10150257
(43) Date of publication of application: 30.05.2012
(73) Proprietor: Hexal Aktiengesellschaft, 83607 Holzkirchen (DE)
(72) Inventor: SCHICKANEDER, Christian, 01309 Dresden (DE); SCHÄFER, Jürgen, 01445 Radebeul (DE)
(74) Representative: Prüfer & Partner GbR European Patent Attorneys
(86) International application number: PCT/EP2011/000018
(87) International publication number: WO 2011/083079

(56) References cited:
- WO-A1-2005/105762
- WO-A1-2008/047104
- WO-A2-2007/134846

## Description

### Field of the invention

The present invention relates in general to the field of organic chemistry, and in particular to the preparation of phenyl derivatives comprising a (1H-1,2,4-triazol-1-yl)alkyl group such as anastrozole (2,2'-(5-((1H-1,2,4-triazol-1-yl)methyl)-1,3-phenylene) bis(2-methyl-propanenitrile).

### Background of the invention

Compounds belonging to the group of phenyl derivatives comprising a (1H-1,2,4-triazol-1-yl)alkyl group may represent valuable nonsteroidal aromatase inhibitors. For example, anastrozole (2,2'-(5-((1H-1,2,4-triazol-1-yl)methyl)-1,3-phenylene) bis(2-methyl-propanenitrile) is in use for treatment of estrogen dependent breast cancer.

EP 0 296 749 A1 discloses (substituted aralkyl)heterocyclic compounds, among others a deuterated anastrozole derivative prepared by two different pathways. In both pathways, the last reaction step is reacting the sodium sodium salt of 1,2,4-triazole with 1-chlorodideuteriomethyl-3,5-bis(2-cyanoisopropyl)toluene. Neither yield nor purity of the product is given.

WO 2005/105762 A1 discloses the preparation of anastrazol wherein 1-bromomethyl-3,5-bis(2-cyanoisopropyl)toluene is reacted with the sodium salt of 1,2,4-triazole. Thereby it was found that the isomer (2,2'-(5-((1H-1,3,5-triazol-1-yl)methyl)-1,3-phenylene) bis(2-methyl-propanenitrile), also called isoanastrozol, is formed as an undesired by-product. Said isoanastrozole was present in anastrazol even after repeated purification by crystallization in an amount of more than 0.5% determined by HPLC. Furthermore, it was found that the content of isoanastrozole impurity can be significantly reduced to below 0.1 % level by purification in form of anastrozole acid addition salts, however, overall yield was quite low.

In Ge, Zemei; Cui, Jialing; Cheng, Tieming; Li, Runtao; Chinese Journal of Medicinal Chemistry, (2003) Vol. 13, No. 3, 146-147, preparation of anastrozole via N-alkylation of 1,2,4-triazole with 1-bromomethyl-3,5-bis(2-cyanoisopropyl)toluene by phase transfer catalysis is reported, wherein xylene is used as the solvent, K₂CO₃/KOH is used as the base, and tetra-n-butylammonium bromide (TBAB) is used as the phase transfer catalyst, and reaction was carried out under reflux conditions. However, this phase transfer catalysis requires harsh reaction conditions in view of reaction temperature and reaction times. The product was purified by repeated re-crystallisations, however there is no information concerning purity detected by HPLC.

WO 2006/108155 A1 describes a process for preparing anastrozole via N-alkylation of 1,2,4-triazole with 1-bromomethyl-3,5-bis(2-cyanoisopropyl)toluene in the presence of a base selected from the group consisting of NaOH, KOH, K₂CO₃ and Na₂CO₃ at temperatures below -20°C. The resulting crude product is purified by aqueous extraction operations, filtration and re-crystallisation, wherein the impurity profile of anastrozole is not more than about 0.16% area by HPLC of isoanastrozole, not more than about 0.14% area by HPLC of 3,5-bis(2-cyanoisopropyl)toluene, and not more than about 0.18% area by HPLC of other impurities.
WO 2007/002722 A1 also discloses a process for preparing anastrozole via N-alkylation of 1,2,4-triazole with 1-bromomethyl-3,5-bis(2-cyanoisopropyl)toluene in the presence of NaOH at temperatures below -20°C, wherein the product has a purity of 99.94% area by HPLC and 0.06% area by HPLC of an impurity other than isoanastrozole. The low reaction temperatures of the processes of WO 2006/108155 A1 and WO 2007/002722 A1 are disadvantageous for industrial application.

WO 2007/039913 discloses a process for preparing anastrozole wherein 1-halogenomethyl-3,5-bis(2-cyanoisopropyl)toluene is reacted with the potassium or sodium salt of 1,2,4-triazole. The purity of the product is 99.9% area by HPLC after purification comprising aqueous work up operations, column chromatography and recrystallisation.

US 2007/0100148 discloses a process for preparing anastrozole wherein 1-bromomethyl-3,5-bis(2-cyanoisopropyl)toluene is reacted with the sodium salt of 1,2,4-triazole. The product is purified by aqueous work-up, filtration over silica gel and recrystallisation. While the thus obtained anastrozole has a puritity of 99.97% by HPLC, yields are quite low.

DE 10 2005 037 484 A1 discloses a process for preparing anastrozole via N-alkylation of 1,2,4-triazole with 1-bromomethyl-3,5-bis(2-cyanoisopropyl)toluene. Purification of the crude product is effected by multiple purification steps, wherein formation of an anastrozole acid addition salt is the key step. Purity of anastrozole obtained by said process is 99.5% by HPLC, while yields are quite low.

WO 2008/047104 A1 discloses a process for preparing anastrozole by alkylation reaction of 3,5-bis(2-cyanoprop-2-yl)benzylbromide with sodium 1,2,4-triazole in a solvent selected from the group consisting of toluene, DMP, acetonitrile and cyclohexane. However, iso-anastrozole impurity formed in the aforementioned alkylation reaction is not separated from the reaction mixture. Rather, a phase transfer catalyst in the form of a quaternary ammonium salt or a crown ether is additionally added to the alkylation reaction mixture, which application requires laborious subsequent purification procedure by means of both chromatography and precipitation/crystallization.

Therefore, the object of the present invention is to provide an improved process for preparing phenyl derivatives comprising a (1H-1,2,4-triazol-1-yl)alkyl group such as anastrozole (2,2'-(5-((1H-1,2,4-triazol-1-yl)methyl)-1,3-phenylene) bis(2-methyl-propanenitrile).

### Summary of the invention

Various aspects, advantageous features and preferred embodiments of the present invention as summarized in the following items, respectively alone or in combination, contribute to solving the object of the invention:
(1) A process for preparing a compound of formula II 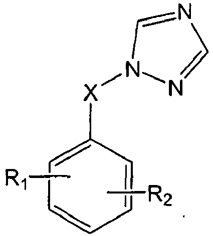 wherein R₁ and R₂ are located at the 3-position and 5-position of the benzene ring of compound of formula I respectively and represent isopropyl substituted in 2-position with cyano, and
   X is methylene,
   wherein a compound of formula I 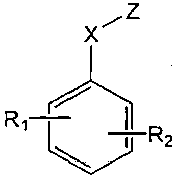 wherein R₁ and R₂ and X are as defined above and Z is a leaving group,
   is reacted with 1,2,4-triazole in a solvent allowing precipitation of a compound of formula III 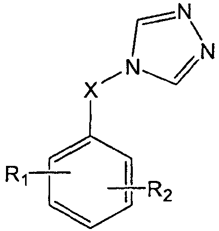 , wherein R₁ and R₂ and X are as defined above and wherein the compound of formula III can be separated from the reaction mixture.

The term "leaving group" is a term commonly used in the art to mean any atom or group of atoms that can depart; typically the leaving group departs with a pair of electrons in a heterolytic bond cleavage that is in the form of anions or neutral molecules during a substitution or displacement reaction. The leaving group may be selected from, without being limited to, halides, such as iodide, bromide and chloride; sulfonates, such as triflates, fluorosulfonates, tosylates, besylates, mesylates; and the like. A preferred leaving group Z is Cl, Br or I.

The reaction can be terminated when conversion to the desired compound is substantially complete. Preferably, conversion is controlled by suitable means, for example by thin layer chromatography.

According to this technical solution of the object of the invention, by selecting a solvent allowing precipitation of compound of formula III, while preferably maintaining the compound of formula II in solution with the solvent, isolation and purification of compound of formula II is significantly simplified by removing the undesired by-product of compound of formula III from the reaction mixture (which has precipitated from the selected solvent), preferably at the end of the reaction. This favours industrial application by providing a robust and competitive process, as well as simplified purification of compound of formula II, while significantly contributing to high yield purity. If desired, a "one pot" concept is made possible since all work-up steps can be easily carried out by, for example, extraction and/or filtration in order to remove undesired by-products, while no laborious chromatography or other purification step is necessary which would have to be carried out outside a reaction system. Compounds of formula II, in particular anastrozole, represent hazardous compounds. Anastrozole is an aromatase inhibitor generally classified as substance of category 4, and for handling such substances, special care has to be taken during industrial manufacturing, particularly regarding health, safety and environmental issues. Thus, the present "one pot" concept contributes to an industrially applicable and competitive process, whilst significantly improving working conditions such that legal, health, environmental and safety (HSE) issues are taken into account.
(2) The process according to item (1), wherein Z is selected from the group consisting of halides and sulfonates, preferably Z is selected from Cl, Br or I, triflates, fluorosulfonates, tosylates, besylates and mesylates, most preferred Z is Br.
(3) The process according to item (1), wherein the solvent allowing precipitation of compound of formula III is selected from aromatic hydrocarbons, ethers and solvent mixtures thereof, provided that said selected solvent precipitates the compound of formula III.
(4) The process according to any one of preceding items, wherein the reaction is carried out in toluene, methyl tert.-butyl ether (MTBE), or a mixture of the solvents with each other or with other solvents.
   According to the preferred embodiments of above items, the formation of compound of formula III 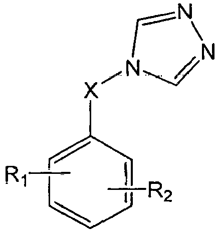 representing an undesired by-product can be substantially reduced, such that exceptionally high yields of the compound of formula II are obtained and purification of the compound of formula II is considerably simplified.
(5) The process according to any one of the previous items for preparing the compound of formula II' 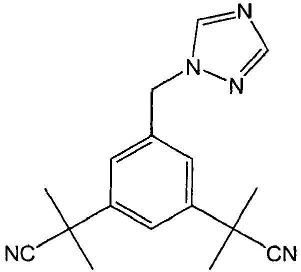 , wherein a compound of formula I' 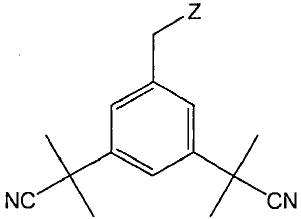 wherein Z is a leaving group,
   is reacted with 1,2,4-triazole in toluene as a solvent.

The reaction can be terminated when conversion to the desired compound is substantially complete. Preferably, conversion is controlled by suitable means, for example by thin layer chromatography. In accordance with this preferred embodiment, a concurrently formed compound of formula III' 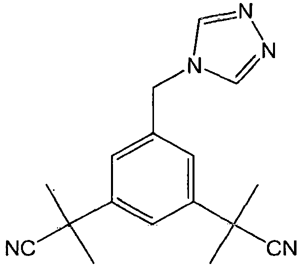 is separated from the reaction mixture, preferably after termination of the reaction. Concerning the description of leaving group Z, reference is made to the description in item (1) above.

According to this embodiment of the invention, by selecting toluene as a particularly suitable solvent, the formation of the compound of formula III' is effectively reduced or minimized during the reaction or is easily removed after its precipitation, which provides for both substantially enhanced yields and exceptional purity of the compound of formula II'. In addition, purification of the compound of formula II' can be markedly simplified by removing the undesired by-product of the compound of formula III' at the end of the reaction. Thus, a "one pot" concept is made possible since all work-up steps can be easily carried out by extraction and/or filtration in order to remove undesired by-products, while no laborious chromatography or other purification step is necessary which would have to be carried outside the reaction vessel. Reference is made to the explanations above regarding HSE constraints of the compound of formula II', representing anastrozole.
(6) The process according to any one of items (1) to (5), wherein the compound of formula III or III' is removed from the reaction mixture at the end of the reaction by filtration.
(7) The process according to any one of items (1) to (6), wherein the reaction is carried out in the presence of a phase transfer catalyst, preferably said phase transfer catalyst is selected from the group of polyethylene glycols, tetraalkyl ammonium salts and tetraalkyl phosphonium salts, more preferably said phase transfer catalyst is selected from the group of polyethylene glycols and tetraalkyl ammonium salts, and in particular said phase transfer catalyst is a polyethylene glycol.
   The term "phase transfer catalyst" as used herein means a catalyst providing for migration or movement of a reactant in a heterologous system from one phase into another phase where reaction can take place. For example, the phase transfer catalyst may provide for migration or movement of a base which is in solid or substantially solid state in the reaction mixture, into the liquid phase of the reaction mixture.
(8) The process according to item (7), wherein the polyethylene glycol phase transfer catalyst is a polyethylene glycol (PEG) phase transfer catalyst having an average molecular weight of about 200 to 800 g/mol, preferably about 300 to 700 g/mol; more preferably about 400 to 600 g/mol; even more preferably, the PEG transfer catalyst is PEG 400 or PEG 600, in particular PEG 600.
(9) The process according to item (7) or (8), wherein the phase transfer catalyst is used in about 0.01 to 0.5 times molar amounts, preferably 0.03 to 0.1 times molar amounts, more preferably 0.04 to 0.09 times molar amounts, and in particular 0.06 to 0.08 times molar amounts relative to compound of formula I.
(10) The process according to any one of the preceding items, wherein said reaction is carried out in the presence of a base selected from the group consisting of linear or branched alkoxides; hydroxides, carbonates or hydrogencarbonates of alkaline metals or alkaline earth metals; preferably KOH, NaOH, NaHCO₃, Na₂CO₃, KHCO₃, K₂CO₃, KO-tert-butyl; more preferably KOH, K₂CO₃, KO-tert-butyl; in particular K₂CO₃.
(11) The process according to any one of the preceding items, wherein the compound of formula I or I', dissolved in a solvent according to item (3) or (4), is added continuously to a stirred mixture comprising 1,2,4-triazole or a salt thereof, a base according to item (10) and a solvent according to item (3).
(12) The process according to item (11), wherein the addition is carried out continuously over a period of about 5 to 240 min, preferably over a period of about 5 to 120 min.
(13) The process according to any one of the preceding items, wherein the reaction is carried out at a temperature of about 20 to 120°C, preferably 40 to 50°C, more preferably 40 to 45°C.
(14) The process according to any one of the preceding items, wherein the reaction is carried out for a time of 0.5 to 12 h, preferably 2.5 to 10 h, more preferably 2.7 to 6.5 h, and in particular 2.8 to 5 h.
(15) The process according to item (1), wherein the compound of formula I is added to a solution comprising 1,2,4-triazole or a salt thereof over a period of about 5 to 240 min, preferably over a period of about 5 to 120 min, the reaction being carried out for a time of 0.5 to 12 h, preferably of 2.5 to 10 h, at a temperature of about 20 to 120°C, preferably 40 to 50°C.
(16) The process according to any one of the preceding items, wherein the ratio of the volume of the solvent employed in said process to the mass of the compound of formula I or I' employed in said process is in a range of 2:1 to 15:1, preferably 3:1 to 12:1, more preferably 5:1 to 11:1, even more preferably 7:1 to 10:1, and in particular 9:1 to 10:1.
(17) The process according to any one of the preceding items, wherein a PEG phase transfer catalyst is used in about 0.01 to 0.5 times molar amounts, preferably 0.03 to 0.1 times molar amounts, more preferably 0.04 to 0.09 times molar amounts, and in particular 0.06 to 0.08 times molar amounts relative to the compound of formula I.
(18) The process according to any one of the preceding items, further comprising a step of forming an acid addition salt of the compound of formula II or of formula II', preferably an HCl addition salt.
(19) The process according to any one of the preceding items, further comprising a step of crystallising or recrystallising the compound of formula II or formula II' or its acid addition salt from a suitable solvent.
(20) The process according to item (19), wherein the compound of formula II or formula II' or its acid addition salt is recrystallised from a solvent comprising or consisting of toluene or isopropanol.
(21) The process according to any one of the preceding items, wherein the compound of formula II or II' or its acid addition salt has a purity of higher than 99.7, such as up to 99.95%, measured by HPLC.
(22) A process for producing a pharmaceutical composition, comprising the steps of:
   i) preparing a compound of formula II or II' or its acid addition salt according to a process of any one of items (1) to (21), and
   ii) mixing said compound of formula II or II' or its acid addition salt with at least one pharmaceutically-acceptable excipient.

The term "pharmaceutical composition" as used herein means a formulation comprising a safe and effective amount of active agent and pharmaceutically-acceptable excipients.

The term "pharmaceutically acceptable excipient" as used herein means any physiologically inert, pharmacologically inactive material known in the art being compatible with the physical and chemical characteristics of the active agent.

### Detailed description of the Invention

The present invention is now described in more detail by referring to further preferred and further advantageous embodiments and examples, which are however presented for illustrative purposes only and shall not be understood as limiting the scope of the present invention.

In order to improve a process for the preparation of a compound of formula II (phenyl derivatives comprising a (1H-1,2,4-triazol-1-yl)alkyl group) and a compound of formula II' (anastrozole, that is 2,2'-(5-((1H-1,2,4-triazol-1-yl)methyl)-1,3-phenylene)bis(2-methyl-propanenitrile), extensive test series were carried out by the inventors to find critical factors that are particularly suited to increase product yields and to decrease the amount of by-products, while significantly simplifying preparation due to beneficial reaction conditions and/or less necessary purification steps.

The compound of formula II' (anastrozole, that is 2,2'-(5-((1H-1,2,4-triazol-1-yl)methyl)-1,3-phenylene)bis(2-methyl-propanenitrile) may be prepared via N-alkylation of 1,2,4 triazole with the a leaving group as described above:

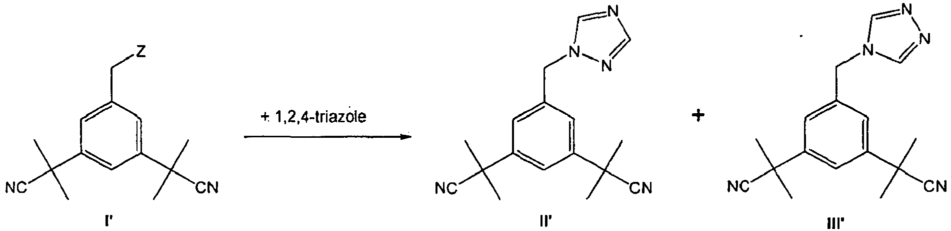

However, in such conventional processes for preparing anastrozole, an isomer compound of formula III', the so-called isoanastrozole, is formed in relatively large amounts. In conventional processes, removal of the compound of formula III' from the crude product of the compound of formula II' is difficult and affords one or multiple laborious subsequent purification step(s), wherein the yield of anastrozole is significantly decreased by said purification step(s). Furthermore, a drawback of some prior art processes is that they require harsh and/or industrially not applicable reaction conditions.

According to the invention, a compound of formula II 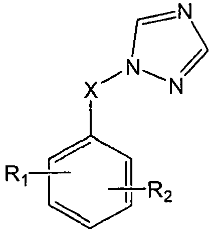 wherein R₁ and R₂ are located at the 3-position and 5-position of the benzene ring of compound of formula I respectively and represent isopropyl substituted in 2-position with cyano, and X ismethylene,
is prepared by a process wherein a compound of formula I 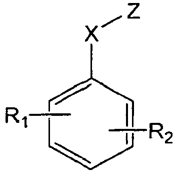 wherein R₁ and R₂ are defined as above, and wherein Z is a leaving group,
is reacted with 1,2,4-triazole in a solvent allowing precipitation of a compound of formula III 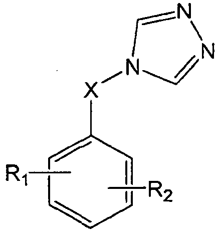 wherein the compound of formula III is precipitated from the selected solvent and separated as precipitated by-product from the reaction mixture, preferably after termination of the reaction.

It was surprisingly found that the invention provides for significantly simplified isolation and purification of the compound of formula II. As a solvent is selected to allow precipitation of the isomer compound of formula III, while preferably maintaining the compound of formula II in solution with the solvent, the undesired by-product compound of formula III can be simply removed at the end of the reaction. Thereby, a "one pot" concept is rendered possible, since all work-up steps can be easily carried out, for example by extraction and/or filtration in order to remove undesired by-products - no laborious chromatography or other purification step is necessary which would have to be carried out outside a reaction system. Compounds of formula II may also represent hazardous compounds. Therefore, the present "one pot" concept contributes to an industrially applicable and competitive process, while working conditions are significantly improved such that legal, health, environmental and safety (HSE) issues are taken into account.

According to aspecific embodiment of the invention, the compound specified above, i.e. having the formula II' 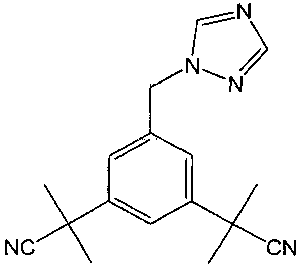 is prepared by a process wherein a compound of formula I' 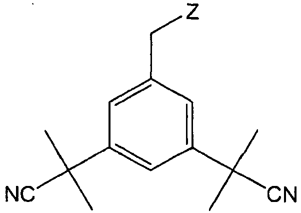 wherein Z is a leaving group,
is reacted with 1,2,4-triazole in toluene as a solvent. In this embodiment, the process is performed such that the compound of formula III' 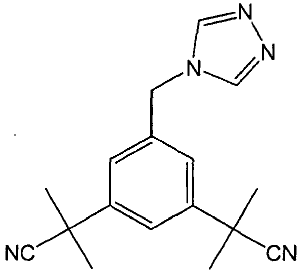 is precipitated and removed from the reaction mixture, preferably after termination of the reaction, and preferably by filtration.

It was found feasible to yield a highly pure compound of formula II' by selecting toluene as the solvent for the reaction in order to effectively reduce or prevent the formation of the compound of formula III' during the reaction. Thereby, the compound of formula II' is obtained in both enhanced yields and exceptional purity. Furthermore, this aspect of the invention also renders possible the aforementioned "one pot" concept.

The compound of formula I' is readily available. For example, it can be prepared as described in WO 2005/105762 A1.

According to a preferred embodiment, the undesired isomer compound of formula III or III' is removed from the reaction mixture at the end of the reaction by filtration.

Preferably, the reaction is carried out in the presence of a phase transfer catalyst, preferably said phase transfer catalyst is selected from the group of polyethylene glycols, tetraalkyl ammonium salts and tetraalkyl phosphonium salts, more preferably said phase transfer catalyst is selected from the group of polyethylene glycols and tetraalkyl ammonium salts, and in particular said phase transfer catalyst is a polyethylene glycol.

It was surprisingly found that carrying out the conversion of compound of formula I' into the compound of formula II' in the presence of a polyethylene glycol (PEG) phase transfer catalyst provides for both mild reaction conditions and high yields. Preferably, the PEG phase transfer catalyst has an average molecular weight of about 200 to 800 g/mol, more preferably about 300 to 700 g/mol; even more preferably about 400 to 600 g/mol; yet even more preferably, the PEG transfer catalyst is PEG 400 or PEG 600, in particular PEG 600. Furthermore, the PEG phase transfer catalyst is suitably used in about 0.01 to 0.5 times molar amounts, preferably 0.03 to 0.1 times molar amounts, more preferably 0.04 to 0.09 times molar amounts, and in particular 0.06 to 0.08 times molar amounts relative to the compound of formula I. Thus, a stable and reliable process is provided. The amount of catalyst relative to the compound of formula I is suitably selected such that the reaction can be smoothly accomplished under mild conditions, while the content of isomer III or III' is surprisingly reduced in an effective manner.

The process according to any one of the aforementioned aspects of the invention is carried out in the above specified solvent.

The content of the isomer compound of formula III 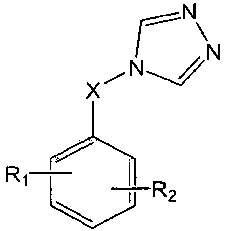 representing an undesired by-product can be substantially reduced, wherein exceptionally high yields of the compound of formula II are obtained and purification of the compound of formula II is considerably simplified. This is enhanced as the reaction is carried out in a solvent chosen to allow precipitation of compound of formula III. Such a preferred solvent is particularly selected from aromatic hydrocarbons, ethers and solvent mixtures of these solvents with each other or with other solvents, provided that said selected solvent precipitates the compound of formula III. To this end, in the process according to this preferred embodiment the reaction is preferably carried out in toluene, methyl tert.-butyl ether (MTBE) or a mixture thereof.

Furthermore, it was surprisingly found that the residual amount of the isomer compound of formula III can be significantly reduced by suitably setting the ratio of the volume of the solvent employed in said process to the mass of the compound of formula I or I' employed in said process. Therefore, the ratio of the volume of the solvent employed in said process to the mass of the compound of formula I or I' employed in said process is preferably set within in a range of 2:1 to 15:1, preferably 3:1 to 12:1, more preferably 5:1 to 11:1, even more preferably 7:1 to 10:1, and in particular 9:1 to 10:1.

Preferably, the aforementioned processes are carried out in the presence of a base selected from the group consisting of linear or branched alkoxides; hydroxides, carbonates or hydrogencarbonates of alkaline metals or alkaline earth metals; preferably KOH, NaOH, NaHCO₃, Na₂CO₃ KHCO₃, K₂CO₃, KO-tert-butyl; more preferably KOH, K₂CO₃, KO-tert-butyl; in particular K₂CO₃.

According to a further preferred embodiment, in the aforementioned processes, the compound of formula I or I' dissolved in one of the aforementioned solvents is added continuously to a stirred mixture comprising 1,2,4-triazole or a salt thereof, one of the aforementioned bases and one of the aforementioned solvents. Preferably, said addition is carried out continuously over a period of about 5 to 240 min, preferably over a period of about 5 to 120 min.

According to a still further preferred embodiment, the aforementioned processes are carried out under at least one condition selected from:
a) the reaction is carried out at a temperature of about 20 to 120°C, preferably 40 to 50°C, more preferably 40 to 45°C;
b) the reaction is carried out for a time of 0.5 to 12 h, preferably 2.5 to 10 h, more preferably 2.7 to 6.5 h, and in particular 2.8 to 5 h..

According to another aspect of the invention, a process for producing a pharmaceutical composition comprises the steps of:
i) preparing a compound of formula II or II' according to any one of preceding items (1) to (22) or a pharmaceutically acceptable salt thereof, and
ii) mixing said compound of formula II or II' with at least one pharmaceutically-acceptable excipient.

Pharmaceutically-acceptable excipients include, but are not limited to, solvents, polymers, plasticizers, fillers, binders, buffer systems, surfactants, dyes or pigments, preservatives and flavouring agents.

According to a preferred embodiment of the invention, a compound of formula II or II' produced by the aforementioned processes is purified by applying at least one of the following steps:
a) washing the combined filtrates obtained from the process with water,
b) converting compound of formula II or II' into its respective acid addition salt,
c) washing an aqueous phase comprising the aforementioned acid addition salt with an organic solvent,
d) converting the acid addition salt of compound of formula II or II' into compound of formula II or II' in free form,
e) washing the organic phase comprising compound of formula II or II' with water, preferably said organic phase is filtered in a subsequent step,
f) treating the organic phase with a discolorating agent,
g) crystallizing compound of formula II or II'.

According to a further preferred embodiment of the invention, steps a) to g) are subsequently carried out.

In step a), a filter residue is washed with a solvent specified above, wherein the filtrate from the reaction mixture and the washing solvent are combined, representing combined filtrates which are washed with water.

Then, in step b), a compound of formula II or II', present in said combined filtrates, is converted to its respective acid addition salt by adding an acid. Preferably, said acid is an organic or inorganic acid, more preferably oxalic acid, hydrochloric acid, hydrobromic acid, even more preferably hydrochloric acid or hydrobromic acid, in particular hydrochloric acid. Furthermore, preferably said acid is added in concentrated form or in the form of a diluted aqueous solution, preferably in the form of a diluted aqueous solution, more preferably in the form of a diluted aqueous solution having a normality in a range of about 0.05 N to 10 N, even more preferably about 0.4 N to 5 N, yet even more preferably about 0.8 N to 3N, and in particular 1 N.

In step c), an aqueous phase comprising the resulting acid addition salt of the compound of formula II or II' is separated from the organic phase, and the aqueous phase is washed with a nonpolar aprotic solvent. Preferably, said nonpolar aprotic solvent is selected from the group of alkanes, alkenes, aromatic hydrocarbons, perhalogenated hydrocarbons, more preferably linear or cyclic alkanes, even more preferably cyclic alkanes, yet even more preferably cyclopentane or cyclohexane, in particular cyclohexane.

Next, in step d), the acid addition salt of the compound of formula II or II' is converted into the compound of formula II by adding a base to the aqueous solution, preferably by adding an inorganic base, more preferably an alkaline metal hydroxide or an alkaline earth metal hydroxide, even more preferably an alkaline metal hydroxide, yet even more preferably NaOH or KOH. Said base is added in concentrated form or in form of a diluted aqueous solution, preferably in the form of a diluted aqueous solution more preferably in the form of a diluted aqueous solution having a normality in a range of about 0.05 N to 10 N, even more preferably about 0.4 N to 5 N, yet even more preferably about 0.8 N to 3N, and in particular 1 N.

In step e), an organic solvent is added prior to or subsequent to addition of the base to the aqueous solution, preferably said solvent is selected from the group consisting of nonpolar aprotic solvents, more preferably from the group consisting of alkanes, alkenes, aromatic hydrocarbons, perhalogenated hydrocarbons, preferably aromatic hydrocarbons or linear or cyclic alkanes, more preferably aromatic hydrocarbons, in particular toluene. Preferably, the organic solvent in which the compound of formula II is dissolved is separated from the aqueous phase and washed with water. More preferably, the resulting organic phase is evaporated and the resulting residue is taken up in a polar aprotic solvent, preferably selected from the group of carbon acid esters, more preferably ethyl acetate, wherein the resulting solution or suspension is stirred in the presence of a filter aid, wherein the solution or suspension is filtered over a bed of said filter aid. Preferably, said filter aid is selected from the group consisting of cellulose filter aid, kieselguhr and silica gel, preferably silica gel. Furthermore, preferably the resulting suspension is stirred at ambient temperature, preferably 10 to 35°C, more preferably 15-25°C.

In step f), an organic phase comprising the compound of formula II or II', preferably an organic phase obtained from the above described step e), is evaporated and the residue is taken up in a polar protic solvent, preferably linear or branched C₁-C₄ alcohols, more preferably 2-propanol, and a discoloration agent is added to the solution or suspension, wherein the resulting mixture is stirred for a predetermined time at a predetermined temperature, and then filtered at the predetermined temperature. Preferably, said discoloration agent is charcoal, preferably activated charcoal. Furthermore, said predetermined temperature is within a range of 15 to 100°C, preferably 20 to 90°C, more preferably 25 to 85°C, in particular at the reflux temperature of the polar protic solvent. After filtration in step f), the filter residue may be washed with a polar protic solvent which is preferably a linear or branched C₁-C₄ alcohol, more preferably 2-propanol, and the filtrate and the washing solvent are combined and represent the combined filtrates.

In step g), the combined filtrates of step e) are cooled and optionally stirred for crystallisation of the compound of formula II or II' at a temperature of about -20 to 30°C, preferably -20 to -10°C, more preferably -10 to -8°C, wherein cooling and optional stirring is preferably carried out for 2 to 3 h. Preferably, the crystals of the compound of formula II which formed in said combined filtrates are separated and washed with a polar protic solvent, preferably linear or branched C₁-C₄ alcohols, more preferably 2-propanol, wherein said protic solvent preferably has a temperature of about -20 to 30°C, preferably -20 to -10°C, more preferably -10 to -8°C. The crystals of the compound of formula II or II' can be dried under normal or reduced pressure, preferably at reduced pressure, at 20°C for 2 h, preferably at 40 to 45 °C for 7 h.

Preferably, the compound of formula II or II' produced by the aforementioned processes and purified by at least one of the above described step a) to g) has a purity of 99.7 to 99.95% measured by HPLC.

The following examples are merely illustrative of the present invention and they should not be considered as limiting the scope of the invention in any way. The examples and modifications or other equivalents thereof will become apparent to those versed in the art in the light of the present entire disclosure.

### Examples

### Methods:

### HPLC purity determinations

### 1. Reagents and reference substances

| | |
|---|---|
| Acetonitrile Far UV | e. g. Fisher art.-no. A/0627/17 |
| Potassium dihydrogen phosphate | e. g. Merck art.-no. 1.04871 |
| ortho-Phosphoric acid 85% | e. g. Merck, art.-no. 1.00563 |
| Water | HPLC quality |

### 2. Solutions to be prepared

### Buffer solution pH 3.0

5.45 g potassium dihydrogen phosphate are dissolved in 1900 ml water. The pH of this solution is adjusted to 3.0 ± 0.05 with ortho-phosphoric acid 85%. Afterwards, the solution is filled-up to 2000.0 ml with water and filtered through a membrane filter (0.45 µm).

### Solvent

Buffer solution pH 3.0 : Acetonitrile in the relation 1 : 1 (V/V)

### Test solution

50.0 mg of substance are dissolved with solvent to 25.0 ml (c = 2 mg/ml). The test solution is prepared once.

### 3. Chromatographic system

| | | |
|---|---|---|
| Column: | stainless steel column (250 x 4.6 mm) filled with octadecylsilyl silica gel for chromatography, e.g. Symmetry C18, 5 µm | |
| Mobile phase A: | Buffer solution pH 3.0 | |
| Mobile phase B: | Acetonitrile | |
| Gradient: | 0 min | 30% B |
| | 30 min | 70% B |
| | 35 min | 70% B |
| | 36 min | 30% B |
| | 42 min | 30% B |
| Flow rate: | 1.2 ml/min | |
| Detection: | UV Detection: 209 nm, BW: 4 nm (Ref.: Off) | |
| Column temperature: | 25°C | |
| Injection volume: | 20 µl | |

### Preparation of compound of formula II' (anastrozole)

### Example 1 and variations thereof: Preparation of compound of formula II' (anastrozole) by using toluene as the solvent and a phase transfer catalyst

### A) Employing toluene in a ratio of approx. 9 : 1 (v/m) relative to compound of formula I'

A solution of **I**' (Z = Br, 328.9 g, 1.0 mol) in toluene (1.32 L) was added to a stirred mixture of K₂CO₃ (179.7 g, **1.3 eq.**)**,** 1,2,4-triazole (72.5 g, **1.05 eq.**), PEG **600** (30.0 g, **0.05 eq.**), and toluene (1.65 L) at 40 - 45 °C. After stirring for **3 h** at **44 - 45 °C,** the mixture (**II**' : **III**' = 8:1, calcd.) was filtered and the residue (**III**', approx. 41 g, 14 %, purity: 81.2 %), was washed with warm toluene (0.5 L). The filter residue was washed with water and dissolved in warm methanol (500 mL). The resulting solution was evaporated to dryness to give 40.9 g (13.9 %) of isomer **III'** (81.2 %, HPLC.). The filtrate was washed with water, obtaining 3.56 L of a solution of compound **II'. HPLC:** 89.4 % of compound **II',** 3.5 % of isomer **III'** (3.5 %), corresponding to an isomeric ratio-of approx. 26:1 (**II**' : **III**'). **Yield:** (91.3 %, calcd.).

In the following Examples 1 B) to D), the ratio (v/m) of volume of toluene relative to mass of compound of formula I' was varied:

### B) Employing toluene in a ratio of approx. 3 : 1 (v/m) relative to compound of formula I'

Similar to example 1, item A). **Yield:** 42.14 g (71.8 %) **HPLC:** 78.1 % of compound **II**', 7.16 % of isomer **III',** corresponding to an isomeric ratio of approx. 11:1 (**II**': **III**').

### C) Employing toluene in a ratio of approx. 7 : 1 (v/m) relative to compound I'

Similar to example 1, item A). **Yield:** 56.91 g (97.0 %), **HPLC:** 86.2 % of compound **II**', 4.78 % of isomer **III',** corresponding to an isomeric ratio of approx. 18:1 (**II**' : **III**').

### D) Employing toluene in a ratio of approx. 10 : 1 (v/m) relative to compound I'

Similar to example 1, item A). **Yield:** 28.88 g (98.4 %), **HPLC:** 88.8 % of compound **II**', 3.25 % of isomer **III',** corresponding to an isomeric ratio of approx. 27:1 (**II**' : **III**').

In the following Examples 1 E) to 1 H), the kind of base and/or the amount of base and/or the reaction temperature was varied:

### E) Employing 2.2 eq of KtOBu

Similar to example 1, item A) but stirring at 9 - 21 °C for 4.5 h. **Yield:** quantitative. **HPLC:** 75.16 % of compound **II',** 3.05 % of isomer **III',** corresponding to an isomeric ratio of approx. 25:1 (**II**' : **III**').

### F) Employing 1.2 eg of KtOBu

Similar to example 1, item E. Yield: quantitative, HPLC: 75.38 % of compound **II',** 3.39 % of isomer **III',** corresponding to an isomeric ratio of approx. 22:1 (**II**' : **III**').

### G) Employing 1.1 eq. of K₂CO₃

Similar to example 1, item A) but employing toluene in a ratio of 6 : 1 (v/m) relative to compound I' and heating at **41 - 60 °C** for **9.5 h. Yield:** 76.94 g (105 %). **HPLC:** 81.9 % of compound II', 8.45 % of isomer III', corresponding to an isomeric ratio of approx. 10:1 (**II' : III**').

### H) Employing 2.2 eq. of K₂CO₃

Similar to example 1, item D). **Yield:** 54.6 g (93.1 %). **HPLC:** 89.2 % of compound **II**', 4.13 % of isomer **III',** corresponding to an isomeric ratio of approx. 22:1 (**II' : III**').

In the following Examples 1 I) to M), the kind of phase transfer catalyst and/or the amount thereof and/or the reaction temperature was varied:

### I) Employing PEG 600, 0.03 eq.

Similar to example 1, item D) but stirring at **52 - 53 °C** for 3.5 h. **Yield:** 27.05 g (92.2 %) **HPLC:** 88.6 % of compound II', 4.43 % of isomer **III',** corresponding to an isomeric ratio of approx. 20:1 (**II'** : **III'**).

### J) Employing PEG 600, 0.1 eq.

Similar to example 1, item D) but stirring at 10 - 20 °C for 4.0 h. **Yield:** 28.94 g (98.6 %) **HPLC:** 89.3 % of compound **II',** 3.54 % of isomer **III',** corresponding to an isomeric ratio of approx. 25:1 (**II'** : **III'**).

### K) Employing PEG 400, 0.05 eq.

Similar to example 1, item D) but employing K₂CO₃ in a ratio of **1.5 eq** and stirring at 40 - 45 °C for 2.75 h. **Yield:** 53.0 g (90.3 %) **HPLC:** 89.0 % of compound **II'**, 3.78 % of isomer **III',** corresponding to an isomeric ratio of approx. 24:1 (**II'** : **III'**).

### L) Employing PEG 400, 0.075 eq.

Similar to example 1, item D) and stirring at 40 - 45 °C for 4 h **Yield:** 50.91 g (86.8 %). **HPLC:** 89.3 % of compound **II'**, 2.42 % of isomer **III'**, corresponding to an isomeric ratio of approx. 37:1 (**II'** : **III'**).

### M) Employing TBAB, 0.062 eq.

Similar to example 1, item G) but employing TBAB (0.062 eq.) as the phase transfer catalyst, a mixture of K₂CO₃ and KOH (1:1, n/n) in a ratio of 1.2 eq. relative to the compound of formula I' and 1.2 eq. of 1,2,4-triazole relative to the compound of formula I'. The mixture was heated at 28 - 34 °C for 4 h. **Yield:** 5.22 g (88.9 %), **HPLC:** 85.4% of compound **II',** 5.5% of isomer **III',** corresponding to an isomeric ratio of approx. 15:1 (**II'** : **III'**).

In the following Examples 1 N) and O), the amount of 1,2,4-triazole was varied:

### N) Employing 1,2,4-triazole, 1.0 eq.

Similar to example 1, item A) but employing K₂CO₃ in a ratio of 1.1 eq., and toluene in a ratio of approx. 6 : 1 (v/m) relative to compound **I'. Yield:** 12.3 g (94.6 %). **HPLC:** 83.4 % of compound **II',** 4.18 % of isomer **III',** corresponding to an isomeric ratio of approx. 20:1 **(II':III').**

### O) Employing 1,2,4-triazole, 1.1 eq.

Similar to example 1, item A) but employing K₂CO₃ in a ratio of 1.3 eq., and toluene in a ratio of approx. 10:1 (v/m) relative to compound **I'. Yield:** 28.10 g (95.8 %) **HPLC:** 85.3 % of compound **II',** 3.27 % of isomer **III',** corresponding to an isomeric ratio of approx. 26:1 **(II':III').**

### Example 2: Preparation of compound of formula II' (anastrozole) by employing MtBE as the solvent and a PEG phase transfer catalyst

A mixture of I' (Z = Br, 19.73 g, 60 mmol), 1,2,4-triazole (4.15 g, 1.0 eq.), K₂CO₃ (9.12 g, 1.1 eq.), PEG 600 (1.86 g, 0.05 eq.) and MtBE (300 mL) was stirred for **6.25** h at reflux (approx. **55 °C**). The mixture was filtered and filter cake was washed twice with MtBE (10 mL). The filtrates were cooled to crystallization and filtered. The crystals were washed four times with MtBE (10 mL) and dried. **HPLC:** isomeric ratio of approx. 27 : 1 **(II'** : **III').**

### Comparative Example 1: Preparation of compound of formula II' (anastrozole) by using TBAB as the phase transfer catalyst and less suitable solvents

### A) Employing Dichloromethane as the solvent

A mixture of 1,2,4-triazole (1.38 g, 1.0 eq.), TBAB (0.4 g, 0.062 eq.) and dichlormethane (30 mL) was charged. Aq. sodium hydroxide (18.87 N , 6.4 mL, 6.0 eq.), **I'** (Z = Br, 6.58 g, 20 mmol) and dichlormethane (10 mL) were added. The mixture was stirred for 2 h at ambient temperature and 2.5 h at reflux. Water (30 mL) was added and the phases were separated. The organic phase was washed with water (3 x 30 mL) and evaporated to give 6.28 g (107.0 %) of an isomeric mixture of compounds **II'** and **III'** (7.3 : 1).

### B) Employing ethyl acetate as the solvent

A mixture of I' (Z = Br, 6.58 g, 20 mmol), 1,2,4-triazole (1.38 g, 1.0 eq.), PEG 600 (0.6 g, 0.05 eq.), K₂CO₃ (6.08 g, 2.2 eq.) and ethyl acetate (40 mL) was stirred for 5 h at ambient temperature and 1.5 h at 31 - 41 °C. The solution was washed with water (3 x) and evaporated to dryness to yield 6.3 g (106.8 %) of a mixture of compounds **II'** and **III'** (4 : 1).

The solvents used in A) and B) are less suitable compared to e.g. toluene, as they are less suitable in forming precipitates of undesired side product (isomer compound of formula III').

### Example 3: Preparation of compound of formula II' (anastrozole) employing a suitable solvent, without using a phase transfer catalyst

Similar to example 1, item G) using toluene as solvent, but employing 1.4 eq. of K₂CO₃ and 1.1 eq. of 1,2,4-triazole, relative to the compound of formula I' and no phase transfer catalyst. The mixture was heated **at 75 °C** for **6 h. Yield:** 69.20 g (94.4 %). **HPLC:** 77.22 % of compound **II',** 5.57 % of isomer **III',** corresponding to an isomeric ratio of approx. 14:1 **(II' : III').**

### Comparative Example 2: Employing different kinds of less suitable solvents and without using a phase transfer catalyst A) Employing acetonitrile as the solvent

A mixture of I' (Z = Br, 3.05 g, 10 mmol), 1,2,4-triazole (3.96 g, 5.7 eq.), and acetonitrile (26 mL) was stirred for 5 h under reflux. The solvent was distilled off under reduced pressure at 70 °C and ethyl acetate (80 mL) was added. The solution was washed with water (3 x), dried over sodium sulphate and evaporated to dryness to yield 2.8 g (88.2 %) of an isomeric mixture of compounds **II'** and **III'** (1 : 1).

### B) Employing acetone as the solvent

A mixture of I' (Z = Br, 3.05 g, 10 mmol), 1 ,2,4-triazole (0,76 g, 1.1 eq.), K₂CO₃ (2,1 g, 1.5 eq.) and acetone (200 mL) was stirred for 2 h **under reflux.** The mixture was allowed to reach room temperature and filtered. The filter residue was washed with acetone and the filtrates were evaporated under reduced pressure to give 3.14 g (99,3 %) of an isomeric mixture of compounds **II'** and **III' (5.1 : 1).**

### C) Employing n-butanol as the solvent

A mixture of 1,2,4-triazole (4.35 g, 1.05 eq.), KOH (5,05 g, 1.5 eq.) and 1-butanol (280 mL) was heated to distillation and 100 mL of solvent was distilled of at 44-57°C under reduced pressure. Then n-butanol (100 mL) and I' (Z = Br, 19.73 g, 60 mmol) were added, the mixture was stirred for 3.5 h at 42 - 48 °C and filtered. The filtrates were washed six times with water (40 mL) and the organic phase was evaporated under reduced pressure to give 18.94 g (107.6 %) of an isomeric mixture of compounds **II'** and **III' 5.5 :** 1).

### D) Employing DMF as the solvent

A solution of I' (Z = Br, 7.23 g, 23.7 mmol) in dimethylformamide (20 mL) was added to a stirred mixture of K₂CO₃ (4.9 g, 1.5 eq.), 1,2,4-triazole (1.8 g, 1.1 eq.) and dimethylformamide (20 mL) at 2 - 3 °C. After heating to ambient temperature over a period of 2 h and stirring for 2 h at 22 - 25 °C, potable water (130 mL) was added and the mixture was extracted twice with ethyl acetate (50 mL). The organic phase was washed three times with potable water (40 mL), dried over sodium sulphate and evaporated to dryness to yield 6.7 g (89.5 %) of an isomeric mixture of compounds **II'** and **III'** (9.7 : 1).

The solvents used in A) to D) are less suitable compared to e.g. toluene, as they are less suitable in forming precipitates of undesired side product (isomer compound of formula III').

### Purification of a compound of formula II' (anastrozole)

### Example 4: Purification of a compound of formula II' (anastrozole) via its acid addition salt

A fraction (784 g) of the solution obtained according to example 1 item A), was extracted with HCl (aq., 1 N, 240 mL). The organic layer was treated with cyclohexane (870 mL), extracted with HCl (aq., 1N, 66 mL, 4 x), and discarded. The combined aqueous phases were washed with toluene (70 mL). The aqueous phase was subsequently treated with toluene (525 mL) and NaOH (aq., conc., 26 mL) with stirring. The organic phase was separated and washed with water. The solvent was removed under reduced pressure and ethyl acetate was added to obtain 640 mL of a turbid solution, containing 57.5 g (97 %) of compound **II'.** The mixture was treated with SiO₂ with stirring and filtered over a bed of SiO₂. The residue was successively washed with ethyl acetate and the combined clear filtrates were taken to dryness under reduced pressure.

### Example 5: Further purification of a compound of formula II' (anastrozole) by recyrstallization

### A) Re-crystallisation from toluene (1:4, m/v)

A fraction (174.2 g) of a crude product obtained similar to example 4 was treated with toluene (700 mL) and activated charcoal. The mixture was heated to reflux and filtered. The filter residue was washed with hot toluene and the filtrate was cooled to approximately -7 °C. The resulting suspension was stirred for 3.5 h at -7 °C and filtered. The crystals were washed once with cold toluene (35 mL), four times with cooled 2-propanol (35 mL) and dried. **Yield:** 159 g (91.3 %) of a colourless crystalline powder. **Mp.:** 83.6 - 84.3 °C. **DSC:** peak at approx. 84 °C. **Purity:** 99.94 % (HPLC). **Assay** (HClO₄): 100.1 %. **Heavy metals:** nmt 20 ppm. **Loss on drying:** 0.10 %. **Sulphated ash:** 0.03 **%. ¹H-NMR** (CDCl₃, 300 MHz, ppm): δ 1.7 (s, 12 H, CH₃), 5.4 (s, 2 H, CH₂), 7.3 (s, 2 H, CH), 7.5 (s, 1 H, CH), 8.0 (s, 1 H CH), 8.2 (s, 2 H, CH). **¹³C-NMR** (CDCl₃, 75 MHz, ppm): δ 29.0, 37.2, 53.0, 122.1, 123.8, 124.3, 136.7, 143.3, 143.4, 152.3. **IR** (KBr, cm⁻¹): 3103, 2985, 2236_{(v·CN)}, 1607, 1502, 1476, 1369, 1359, 876. **GC/MS** (EI, m/z): 293.1 [M]⁺.

### B) Recrystallisation from 2-propanol (1:8, m/v)

A fraction (43.5 g) of the residue obtained according to example 4 was treated with 2-propanol (370 mL) and activated charcoal. The mixture was heated to reflux and filtered. The filter residue was washed with hot 2-propanol and the filtrate was cooled to approximately - 8 °C. The resulting suspension was stirred for 2.5 h at -8 to -10 °C and filtered. The crystals were separated by filtration, washed three times with 12.5 mL of cooled 2-propanol and dried. **Yield:** 40.1 g (92 %) of a colourless crystalline powder (mp. 84 °C). **Purity:** 99.85 % (HPLC). **DSC:** peak at approx. 84 °C.

### C) Re-crystallisation from 2-propanol (1:4, m/v)

Similar to example 5, item B) but employing 2-propanol in a ratio of 1:4 (m/v) relative to the distillation residue obtained according to example 1, item A). **Yield:** 236.9 g (94.8 %).

### D) Re-crystallisation from 2-propanol (1:2, m/v)

Similar to example 4, item B) but employing 2-propanol in a ratio of 2:1 (m/v) relative to the distillation residue obtained according to example 1, item A). **Yield:** 74.0 g (95.2 %). **Mp.:** 83.9 - 84.3 °C. **DSC:** peak at approx. 85 °C. **Purity:** 99.93 % (HPLC). **Heavy metals:** nmt 20 ppm. **Loss on drying:** 0.04 %. **Sulphated ash:** 0.02 %. **Assay:** (HClO₄): 100.3 %.

### E) Re-crystallisation from 1-propanol (1:4, m/v)

Similar to example 4, item B) but employing 1-propanol in a ratio of 1:4 (m/v) relative to the distillation residue obtained according to example 1, item A). **Yield:** 8.56 g (85.6 %). **Purity:** 99.96 % (HPLC).

### F) Re-crystallisation from cyclohexane

Compound **II'** (0.1 g) was dissolved in cyclohexane (20.3 mL) at reflux temperature. The mixture was cooled slowly until crystallization was observed. The crystals were separated by filtration and dried at 40 °C under reduced pressure. **Yield:** 0.08 g (80 %)

### G) Re-crystallisation from cyclohexane and ethyl acetate (1:1)

Compound **II'** (5 g) was dissolved in a mixture of ethyl acetate and cyclohexane (10 mL / 10 mL) at reflux temperature and cooled slowly until crystallization was observed. The crystals were separated by filtration and dried under reduced pressure. **Yield:** 3.15 g (63.0 %)

### H) Re-crystallisation from chloroform

Compound **II'** (2 g) was dissolved in chloroform (10 mL) at ambient temperature and the solvent was slowly evaporated. **Yield:** 1.65 g (82.5 %).

## Claims

1. A process for preparing a compound of formula II 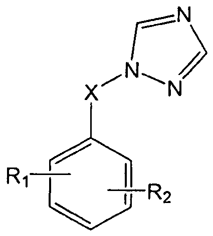 wherein R₁ and R₂ are located at the 3-position and 5-position of the benzene ring of compound of formula I respectively and represent isopropyl substituted in 2-position with cyano, and
X is methylene,
wherein a compound of formula I 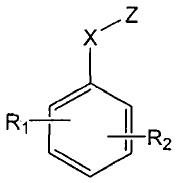 wherein R₁ and R₂ and X are as defined above and Z is a leaving group, is reacted with 1,2,4-triazole in a solvent allowing precipitation of a compound of formula III 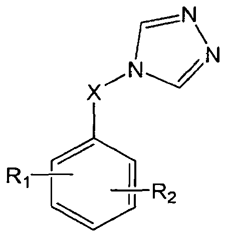 , wherein R₁ and R₂ and X are as defined above, wherein the compound of formula III is precipitated from the selected solvent and separated as precipitated undesired byproduct from the reaction mixture.

2. The process according to claim 1, wherein
Z is selected from Cl, Br, I, triflates, fluorosulfonates, tosylates, besylates and mesylates;
preferably Z is Br.

3. The process according to claim 1, wherein the solvent allowing precipitation of the compound of formula III is selected from aromatic hydrocarbons, ethers and solvent mixtures thereof.

4. The process according to any one of claims 1 to 3, wherein the reaction is carried out in a solvent selected from toluene, methyl tert.-butyl ether (MTBE) and solvent mixtures thereof, preferably toluene.

5. The process according to any one of the previous claims, wherein precipitated compound of formula III 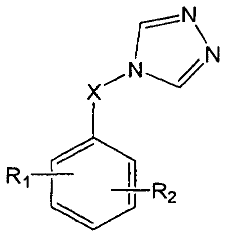 is removed from the reaction mixture at the end of the reaction by filtration.

6. The process according to claim 1, wherein the reaction is carried out in the presence of a phase transfer catalyst selected from the group of polyethylene glycols, tetraalkyl ammonium salts and tetraalkyl phosphonium salts, more preferably said phase transfer catalyst is selected from the group of polyethylene glycols and tetraalkyl ammonium salts, and in particular said phase transfer catalyst is a polyethylene glycol.

7. The process according to claim 6, wherein the polyethylene glycol phase transfer catalyst is a polyethylene glycol (PEG) phase transfer catalyst having an average molecular weight of about 200 to 800 g/mol, preferably about 300 to 700 g/mol; more preferably about 400 to 600 g/mol; even more preferably, the PEG transfer catalyst is PEG 400 or PEG 600, in particular PEG 600.

8. The process according to claim 6 or 7, wherein a PEG phase transfer catalyst is used in about 0.01 to 0.5 times molar amounts, preferably 0.03 to 0.1 times molar amounts, more preferably 0.04 to 0.09 times molar amounts, and in particular 0.06 to 0.08 times molar amounts relative to the compound of formula I.

9. The process according to any one of the preceding claims, wherein said process is carried out in the presence of at least one base selected from the group consisting of linear or branched alkoxides; and hydroxides, carbonates or hydrogencarbonates of alkaline metals or alkaline earth metals; preferably the at least one base is KOH, NaOH, NaHCO₃, Na₂CO₃, KHCO₃, K₂CO₃, KO-tert-butyl; more preferably KOH, K₂CO₃, KO-tert-butyl; in particular K₂CO₃.

10. The process according to any one of the preceding claims, wherein at least one of the following conditions (a) to (d) is applied:
(a) the ratio of the volume of the solvent employed in said process to the mass of the compound of formula I employed in said process is in a range of 2:1 to 15:1, preferably 3:1 to 12:1, more preferably 5:1 to 11:1, even more preferably 7:1 to 10:1, and in particular 9:1 to 10:1;
(b) the compound of formula I, dissolved in said solvent, is added continuously to a stirred mixture comprising 1,2,4-triazole or a salt thereof, a base according to claim 9 and said solvent; wherein preferably addition is carried out continuously over a period of about 5 to 240 min, more preferably over a period of about 5 to 120 min;
(c) the reaction is carried out at a temperature of about 20 to 120°C, preferably 40 to 50°C;
(d) the reaction is carried out for a time of 0.5 to 12 h.

11. The process according to any one of the preceding claims, further comprising a step of forming an acid addition salt of the compound of formula II, preferably an HCl addition salt.

12. The process according to any one of the preceding claims, further comprising a step of crystallising or recrystallising the compound of formula II or its acid addition salt from a suitable solvent.

13. The process according to any one of the preceding claims, wherein compound of formula II has a purity of higher than 99.7 measured by HPLC.

14. A process for producing a pharmaceutical composition, comprising the steps of:
i) carrying out a process according to any one of claims 1 to 13 to prepare a compound of formula II or its acid addition salt, and
ii) mixing said compound of formula II or its acid addition salt with at least one pharmaceutically-acceptable excipient.

## Patentansprüche

1. Verfahren zur Herstellung einer Verbindung der Formel II 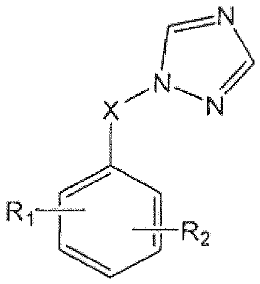 wobei R₁ und R₂ jeweils an der 3-Position und 5-Position des Benzolrings der Verbindung der Formel I lokalisiert sind und mit Cyano substituiertes Isopropyl in 2-Position darstellen, und
X Methylen ist,
wobei eine Verbindung der Formel 1 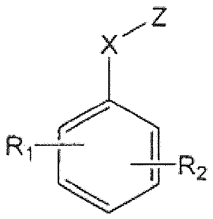 wobei R₁ und R₂ und X wie oben definiert sind und Z eine Abgangsgruppe ist, mit 1,2,4-Triazol in einem Lösungsmittel, welches Fällung
einer Verbindung der Formel III
erlaubt, reagiert wird, 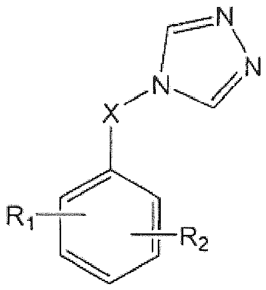 wobei R₁ und R₂ und X wie oben definiert sind, wobei die Verbindung der Formel III vom ausgewählten Lösungsmittel gefällt wird und als ausgefälltes unerwünschtes Nebenprodukt von der Reaktionsmischung abgetrennt wird.

2. Verfahren gemäß Anspruch 1, wobei
Z aus Cl, Br, I, Triflaten, Fluorsulfonaten, Tosylaten, Besylaten und Mesylaten ausgewählt ist;
vorzugsweise Z Br ist.

3. Verfahren gemäß Anspruch 1, wobei das Lösungsmittel, welches die Fällung der Verbindung der Formel III erlaubt, aus aromatischen Kohlenwasserstoffen, Ethern, und Lösungsmittelmischungen von diesen ausgewählt ist.

4. Verfahren gemäß einem der Ansprüche 1 bis 3, wobei die Reaktion in einem Lösungsmittel, welches aus Toluol, Methyl-tert-butylether (MTBE) und Lösungsmittelmischungen von diesen ausgewählt ist, ausgeführt wird, vorzugsweise Toluol.

5. Verfahren gemäß einem der vorhergehenden Ansprüche, wobei die ausgefällte Verbindung der Formel III 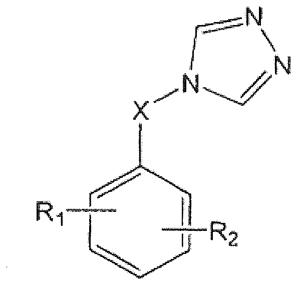 aus der Reaktionsmischung am Ende der Reaktion durch Filtration entfernt wird.

6. Verfahren gemäß Anspruch 1, wobei die Reaktion in Gegenwart von einem Phasentransferkatalysator ausgeführt wird, welcher aus der Gruppe von Polyethylenglykolen, Tetraalkyl-Ammonium-Salzen und Tetraalkyl-Phosphonium-Salzen ausgewählt ist, weiter bevorzugt der Phasentransferkatalysator aus der Gruppe von Polyethylenglykolen und Tetraalkyl-Ammonium-Salzen ausgewählt ist, und insbesondere der Phasentransferkatalysator ein Polyethylenglykol ist.

7. Verfahren gemäß Anspruch 6, wobei der Polyethylenglykol-Phasentransferkatalysator ein Polyethylenglykol (PEG)-Phasentransferkatalysator ist, welcher ein durchschnittliches Molekulargewicht von etwa 200 bis 800 g/mol besitzt, vorzugsweise etwa von 300 bis 700 g/mol; weiter bevorzugt etwa von 400 bis 600 g/mol; noch weiter bevorzugt der PEG-Transferkatalysator PEG 400 oder PEG 600, insbesondere PEG 600 ist.

8. Verfahren gemäß Anspruch 6 oder 7, wobei ein PEG-Phasentransferkatalysator in etwa 0,01 bis 0,5-fachen molaren Mengen verwendet wird, vorzugsweise 0,03 bis 0,1-fachen molare Mengen, weiter bevorzugt 0,04 bis 0,9-fachen molaren Mengen, und insbesondere 0,06 bis 0,08-fachen molaren Mengen bezogen auf die Verbindung der Formel I.

9. Verfahren gemäß einem der vorhergehenden Ansprüche, wobei das Verfahren in Gegenwart von mindestens einer Base ausgeführt wird, ausgewählt aus der Gruppe, welche aus linearen oder verzweigten Alkoxiden; und Hydroxiden, Carbonaten oder Hydrogencarbonaten von Alkalimetallen oder Erdalkalimetallen besteht; vorzugsweise die mindestens eine Base KOH, NaOH, NaHCO₃, Na₂CO₃, KHCO₃, K₂CO₃, KO-tert.-Butyl ist; weiter bevorzugt KOH, K₂CO₃, KO-tert.-Butyl; insbesondere K₂CO₃.

10. Verfahren gemäß einem der vorhergehenden Ansprüche, wobei mindestens eine der folgenden Bedingungen (a) bis (d) angewendet wird:
(a) das Verhältnis des Volumens des Lösungsmittels, welches im Verfahren verwendet wird, zur Masse der Verbindung der Formel I, welche im Verfahren benutzt wird, ist in einem Rahmen von 2:1 bis 15:1, vorzugsweise 3:1 bis 12:1, weiter bevorzugt 5:1 bis 11:1, noch weiter bevorzugt 7:1 bis 10:1, und insbesondere 9:1 bis 10:1;
(b) die Verbindung der Formel I, gelöst im Lösungsmittel, wird kontinuierlich zu einer gerührten Mischung, welche 1,2,4-Triazol oder ein Salz davon, eine Base gemäß Anspruch 9 und das Lösungsmittel umfasst, hinzugegeben; wobei die Hinzugabe vorzugsweise kontinuierlich über eine Zeitspanne von ungefähr 5 bis 240 Minuten, weiter bevorzugt über eine Zeitspanne von ungefähr 5 bis 120 Minuten ausgeführt wird;
(c) die Reaktion wird bei einer Temperatur von ungefähr 20° bis 120°C, vorzugsweise 40° bis 50°C ausgeführt;
(d) die Reaktion wird über eine Zeit von 0,5 bis 12 Stunden ausgeführt.

11. Verfahren gemäß einem der vorhergehenden Ansprüche, weiterhin umfassend einen Schritt der Bildung eines Säureadditionssalzes der Verbindung der Formel II, vorzugsweise ein HCl-Additionssalz.

12. Verfahren gemäß einem der vorhergehenden Ansprüche, weiterhin umfassend einen Schritt der Kristallisation oder Rekristallisation der Verbindung der Formel II oder ihres Säureadditionssalzes aus einem geeigneten Lösungsmittel.

13. Verfahren gemäß einem der vorhergehenden Ansprüche, wobei die Verbindung der Formel II eine Reinheit von mehr als 99,7, gemessen durch HPLC, besitzt.

14. Verfahren zur Herstellung einer pharmazeutischen Zusammensetzung, umfassend die Schritte:
i) Ausführen eines Verfahrens gemäß einem der Ansprüche 1 bis 13, um eine Verbindung der Formel II oder ihres Säureadditionssalzes herzustellen, und
ii) Mischen der Verbindung der Formel II oder ihres Säureadditionssalzes mit mindestens einem pharmazeutisch hinnehmbaren Hilfsstoff.

## Revendications

1. Procédé de préparation d'un composé de formule II 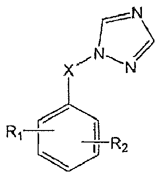 dans lequel R₁ et R₂ sont situés respectivement en position 3 et en position 5 du noyau benzène du composé de formule I et représentent un groupe isopropyle substitué en position 2 par un groupe cyano, et
X est un groupe méthylène,
dans lequel un composé de formule I 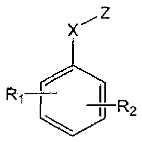 dans laquelle R₁, R₂ et X sont tels que définis ci-dessus et Z est un groupe partant,
est mis à réagir avec un 1,2,4-triazole dans un solvant permettant la précipitation d'un composé de formule III 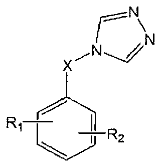 dans lequel R₁, R₂ et X sont tels que définis ci-dessus, dans lequel le composé de formule III est précipité à partir du solvant choisi et est séparé sous forme d'un sous-produit indésirable précipité du mélange réactionnel.

2. Procédé selon la revendication 1, dans lequel
Z est choisi parmi Cl, Br, I, les triflates, les fluorosulfonates, les tosylates, les bésylates et les mésylates ;
de préférence Z est Br.

3. Procédé selon la revendication 1, dans lequel le solvant permettant la précipitation du composé de formule III est choisi parmi les hydrocarbures aromatiques, les éthers et les mélanges de solvants de ceux-ci.

4. Procédé selon l'une quelconque des revendications 1 à 3, dans lequel la réaction est réalisée dans un solvant choisi parmi le toluène, le méthyl-tert-butyléther (MTBE) et des mélanges de solvants de ceux-ci, de préférence le toluène.

5. Procédé selon l'une quelconque des revendications précédentes, dans lequel le composé de formule III précipité 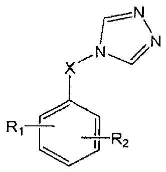 est éliminé du mélange réactionnel à la fin de la réaction par filtration.

6. Procédé selon la revendication 1, dans lequel la réaction est réalisée en présence d'un catalyseur de transfert de phase choisi dans le groupe des polyéthylène glycols, des sels de tétra-alkyl-ammonium et des sels de tétra-alkyl-phosphonium, de manière davantage préférée, ledit catalyseur de transfert de phase est choisi dans le groupe des polyéthylène glycols et des sels de tétra-alkyl-ammonium, et en particulier, ledit catalyseur de transfert de phase est un polyéthylène glycol.

7. Procédé selon la revendication 6, dans lequel le catalyseur de transfert de phase de type polyéthylène glycol est un catalyseur de transfert de phase de type polyéthylène glycol (PEG) ayant une masse moléculaire moyenne d'environ 200 à 800 g/mol, de préférence d'environ 300 à 700 g/mol, de manière davantage préférée d'environ 400 à 600 g/mol, de manière encore davantage préférée le catalyseur de transfert de type PEG est le PEG 400 ou le PEG 600, en particulier le PEG 600.

8. Procédé selon la revendication 6 ou 7, dans lequel un catalyseur de transfert de phase de type PEG est utilisé en des quantités molaires d'environ 0,01 à 0,5 fois, de préférence des quantités molaires de 0,03 à 0,1 fois, de manière davantage préférée des quantités molaires de 0,04 à 0,09 fois, et en particulier des quantités molaires de 0,06 à 0,08 fois, par rapport au composé de formule I.

9. Procédé selon l'une quelconque des revendications précédentes, dans lequel ledit procédé est réalisé en présence d'au moins une base choisie dans le groupe constitué des alcoxydes linéaires ou ramifiés ; et des hydroxydes, des carbonates ou des hydrogénocarbonates de métaux alcalins ou de métaux alcalino-terreux ; de préférence l'au moins une base est KOH, NaOH, NaHCO₃, Na₂CO₃, KHCO₃, K₂CO₃, KO-tert-butyle ; de manière davantage préférée KOH, K₂CO₃, KO-tert-butyle ; en particulier K₂CO₃.

10. Procédé selon l'une quelconque des revendications précédentes, dans lequel l'une au moins des conditions (a) à (d) suivantes est appliquée :
(a) le rapport du volume du solvant employé dans ledit procédé à la masse du composé de formule I employé dans ledit procédé est dans une plage de 2:1 à 15:1, de préférence de 3:1 à 12:1, de manière davantage préférée de 5:1 à 11:1, de manière encore davantage préférée de 7:1 à 10:1, et en particulier de 9:1 à 10:1 ;
(b) le composé de formule I, dissous dans ledit solvant, est ajouté en continu à un mélange agité comprenant du 1,2,4-triazole ou un sel de celui-ci, une base selon la revendication 9 et ledit solvant ; dans lequel de préférence l'addition est réalisée en continu sur une période d'environ 5 à 240 min, de manière davantage préférée sur une période d'environ 5 à 120 min ;
(c) la réaction est réalisée à une température d'environ 20 à 120 °C, de préférence de 40 à 50 °C ;
(d) la réaction est réalisée pendant une durée de 0,5 à 12 h.

11. Procédé selon l'une quelconque des revendications précédentes, comprenant en outre une étape de formation d'un sel d'addition d'acide du composé de formule II, de préférence d'un sel d'addition de HCl.

12. Procédé selon l'une quelconque des revendications précédentes, comprenant en outre une étape de cristallisation ou de recristallisation du composé de formule II ou de son sel d'addition d'acide dans un solvant convenable.

13. Procédé selon l'une quelconque des revendications précédentes, dans lequel le composé de formule II a une pureté supérieure à 99, 7 mesurée par HPLC.

14. Procédé de production d'une composition pharmaceutique, comprenant les étapes de :
i) réalisation d'un procédé selon l'une quelconque des revendications 1 à 13 pour préparer un composé de formule II ou son sel d'addition d'acide, et
ii) mélange dudit composé de formule II ou de son sel d'addition d'acide avec au moins un excipient pharmaceutiquement acceptable.
